# EUROPEAN PATENT SPECIFICATION

(11) **EP 4 651 792 B1**
(45) Date of publication and mention of the grant of the patent: **13.05.2026**
(21) Application number: 24738213.8
(22) Date of filing: 18.06.2024
(51) Int. Cl.: A61B 5/00, A61B 5/28, A61B 5/282, A61B 5/332, A61B 5/335, A61B 5/318, A61B 5/257

(54) **A FULLY-DISPOSABLE ELECTROCARDIOGRAPH ECG RECORDER AND AN ECG MONITORING SYSTEM**
VOLLSTÄNDIG WEGWERFBARER ELEKTROKARDIOGRAFISCHER EKG-RECORDER UND EKG-ÜBERWACHUNGSSYSTEM
ENREGISTREUR D'ÉLECTROCARDIOGRAMME (ECG) ENTIÈREMENT JETABLE ET SYSTÈME DE SURVEILLANCE D'ECG

(30) Priority: 03.04.2024 CN 202420675540 U; 03.04.2024 CN 202420675819 U; 03.04.2024 CN 202410397757; 03.04.2024 CN 202420675683 U; 03.04.2024 CN 202410397461
(43) Date of publication of application: 26.11.2025
(73) Proprietor: Thoth (Suzhou) Medical Technology Co., Ltd., Suzhou, Jiangsu 215100 (CN)
(72) Inventor: GAO, Linming, Suzhou, Jiangsu 215100 (CN); FAN, Zhequan, Suzhou, Jiangsu 215100 (CN); FU, Jianfa, Suzhou, Jiangsu 215100 (CN); HUANG, Huimei, Suzhou, Jiangsu 215100 (CN)
(74) Representative: Huang, Liwei
(86) International application number: PCT/CN2024/099806
(87) International publication number: WO 2025/208715

(56) References cited:
- WO-A1-2018/153188
- WO-A1-2021/136246
- CN-U- 203 436 325
- CN-U- 205 493 818
- US-A1- 2014 031 663
- US-A1- 2015 087 950
- US-A1- 2016 089 049
- US-A1- 2022 304 628

## Description

The present application claims the priority of the Chinese patent applications filed to the Chinese Patent Office on April 3, 2024, with application numbers 202410397461.0, 202420675540.9, 202410397757.2, 202420675819.7, 202420675683.X, and titled as "A fully-disposable ECG recorder and monitoring system", "A signal detecting device", "An ECG recorder", "A host structure and a signal detecting device", "A structure for signal acquisition and a patch of detecting device having the same", respectively.

### Technical Field

The present invention relates to the field of wearable medical devices, and specifically to a fully-disposable electrocardiograph ECG recorder and an ECG monitoring system.

### Background Art

Arrhythmia refers to abnormal frequency or rhythm of heart beats caused by abnormal cardiac activities in origin or in conduction. It belongs to an important group of cardiovascular diseases, which may occur alone or in conjunction with other cardiovascular diseases. Arrhythmia may cause various types of symptoms, however it is worth noting that not all patients with arrhythmia will show symptoms, and in some people, arrhythmias may be discovered only incidentally/occasionally during a physical exam or an ECG. For occasional arrhythmias, a rapid and reliable monitoring becomes difficult.

Holter ECG monitoring is a common method for monitoring arrhythmia. It uses a dynamic ECG recorder to record the whole process of a patient's cardiac activity for 24 hours or longer in daily life, and then analyzes and processes the data with the help of a computer. This detecting method can capture arrhythmias and myocardial ischemia, which are not easy to be detected in a traditional ECG, and thus provide important objective basis for clinical diagnosis/treatment and judgment to evaluate the therapeutic efficacy.

The current dynamic ECG recorder comprises a reusable module for recording physiological data, and a disposable flexible patch configured to be attached to human skin. This type of recorder is also called a semi-disposable ECG recorder or a split-type ECG recorder. The semi-disposable ECG recorder comprises a detachable recording module for physiological data, the recording module has electrical contacts to be connected to the flexible electrical path in the flexible patch. When in use, the recording module for physiological data is connected to the flexible patch, and the flexible patch is attached to the surface of human skin, so as to acquire, process and store ECG signals; after the signal acquisition is completed, the recording module for physiological data is taken out, and the doctor analyzes the data in the recording module; thereafter, the recording module can be disinfected for the next use.

The above-mentioned semi-disposable ECG recorder needs a process of assembling the recording module with the flexible patch, a process of disassembling the recording module from the flexible patch, as well as a process of reusing the recording module. Thus, it will bring problems in use convenience and information security. Besides, the connection of electrical contacts between the recording module and the flexible patch will also bring potential risks in connection stability.

In order to solve the problem of semi-disposable ECG recorders in use convenience, a fully-disposable ECG recorder, which can be completely discarded after use, is proposed.

However, the fully-disposable ECG recorders in the prior art often have problems such as complex processes and a low yield rate in manufacturing.

US2016/089049A1discloses a device for long term sensing and recording electrocardiogram, wherein the housing body includes an upper body and a lower body sealed to form an internal space for enclosing the processing circuit. The middle portion of the water-proof film is sandwiched between the upper body and the lower body. The flexible attach layer is beneath the water-proof film, and the electrodes penetrate the housing body to electrically connect the processing circuit.

### Summary of the disclosure

The technical problem to be solved by the present disclosure is to overcome the problems of the semi-disposable ECG recorders in the prior art such as poor use convenience, poor information security, poor connection stability, etc., and to overcome the problems of the fully-disposable ECG recorders in the prior art such as complex processes and a low yield rate in manufacturing, and to provide a new fully-disposable ECG recorder.

The present invention is set out in the appended set of claims.

When the fully-disposable ECG recorder is attached to the human body, the lower side of the sticky layer is in contact with the human body.

Optionally, an upper release film and a lower release film are provided on the upper and lower sides of the flexible patch, respectively.

Optionally, the fully-disposable ECG recorder further comprises a battery, the signal processing unit comprises a circuit board and a chip on the circuit board, and the circuit board has an interface for connecting with the connecting end of the flexible electrical path.

Optionally, an accommodating structure is provided in the shell, and the accommodating structure is used for accommodating battery and/or the circuit board.

For example, the accommodating structure comprises multiple claws, and the claws are arranged to surround the battery.

Optionally, the antenna is integrated with the circuit board.

Optionally, the limiting member comprises/is a strip-shaped protrusion; the limiting member comprises a first surface facing the battery and a second surface opposite to the first surface, and the antenna is assembled on the second surface.

Optionally, the signal processing unit comprises a connecting member; the connecting member comprises a first contact and a second contact; the battery comprises a first electrode and a second electrode; the first electrode and the second electrode are electrically connected to the first contact and the second contact through a first connector and a second connector, respectively.

Optionally, the insulating pull tab is located between the first electrode and the first connector, or the insulating pull tab is located between the second electrode and the second, or the insulating pull tab is located between the first connector and the first contact, or the insulating pull tab is located between the second connector and the second contact.

Optionally, a positioning structure is provided in the first shell part, and the positioning structure comprises a first positioning wall and a second positioning wall which are arranged opposite to each other; two spring connectors are located in a gap between the first positioning wall and the second positioning wall.

Optionally, the insulating pull tab is located between the spring connector and the first connector, and/or the insulating pull tab is located between the spring connector and the second connector.

Optionally, the first connector has a spring sheet; the first electrode is located on a bottom surface of the battery, the spring sheet is located between the battery and the first shell part; after the insulating pull tab is pulled out, the spring sheet abuts against the first electrode on the bottom surface of the battery.

Optionally, a recess is provided on the inner surface of the first shell part, so as to accommodate at least a part of the spring sheet.

Optionally, the portion of the connecting end entering the shell is bent for a first time for extending parallel to the bottom surface of the battery, and then is bent for the second time at the edge of the battery / circuit board for extending upward, and then is bent for the third time toward the interface.

Optionally, the inner surface of the shell is provided with a strip-shaped groove, the portion of the connecting end extending parallel to the bottom surface of the battery is accommodated in the strip-shaped groove.

Optionally, at least a portion of the connecting end is fixed to the shell by injection molding.

Optionally, the second shell part and the first shell part are connected by snap-fit joint, especially inseparable snap-fit joint.

The present disclosure also provides an ECG monitoring system, comprising the ECG recorder; a server, configured to receive ECG data from the ECG recorder, and the server is configured to process the ECG data after receiving the ECG data from the ECG recorder.

Optionally, the ECG monitoring system further comprises a client terminal, wherein the client terminal is in communication with the ECG recorder and/or the server, and the client terminal is configured to display the ECG data to the user.

In another aspect, the present disclosure provides a signal acquisition structure, comprising a flexible (printed) electrical path and a sticky layer. The flexible electrical path comprises a substrate layer and an electric layer located on the substrate layer; the electric layer comprises multiple conductive wires and multiple electrodes respectively connected to the plurality of conductive wires. At one end, the multiple conductive wires are arranged adjacent to each other, so as to form a connecting end of the flexible electrical path. At the other end, the multiple conductive wires are respectively connected with the multiple electrodes. The adhesive layer covers a portion of the flexible electrical path and does not cover any electrode.

In another aspect, the present disclosure provides a host structure, comprising a shell, a signal processing module located in the shell, and a signal transmitting part (such as an antenna) connected to the signal processing module. The signal transmitting part is located in the shell. The host structure is provided with a limiting part located in the shell, the limiting part is used to limit the position of the signal transmitting part. For example, the limiting part may be in contact with the signal transmitting part.

The fully-disposable ECG recorder and the ECG monitoring system provided by the present disclosure have the following advantages:
1. The fully-disposable ECG recorder provided in the present disclosure has a shell and a flexible patch which are fixed together. During use, the ECG recorder does not need assembling/disassembling, and after use, the ECG recorder can be completely discarded, thereby improving the convenience in use.
2. The connecting end of the flexible electrical path extends from the outside of the shell into the closed space inside the shell, and is connected to the signal processing unit in the enclosed space. Thereby, it can overcome the problems in the prior art, which are caused by the connection position being arranged outside the shell, such as poor connection stability, susceptibility to external environmental influences such as temperature and humidity.
3. The signal processing unit is accommodated in a closed shell, and the sealing signal processing unit and other electronic components can be achieved without using a process of heat laminating or heat sealing. Unlike heat laminating/sealing, the manufacturing method of the present disclosure will not cause damage to the electronic elements inside the recorder. The manufacturing process is simple and the yield rate of qualified products is high.
4. In the fully-disposable ECG of the present disclosure, an first opening is provided on the lower surface of the shell, and the connecting end of the flexible electrical path can be bent upward, so as to pass through the first opening and extend into the enclosed space in the shell. Thereby, it facilitates the assembly process and improving the yield rate of qualified products.
5. The fully-disposable ECG recorder provided in the present disclosure has an upper shell and a lower shell which are separated before assembly and are inseparable after assembly. During the assembly from bottom to top, the flexible electrical path enters the lower shell space, and then is connected to the signal processing unit, and then the upper shell covers the lower shell to form a closed space. Thereby, it facilitates the assembly process and improving the yield rate of qualified products.
6. In the fully-disposable ECG recorder provided in the present disclosure, a reinforcing sheet may be provided on the connecting end of the flexible electrical path. The existence of the reinforcing sheet facilitates the assembly process during manufacture, which makes it easier to insert the connecting end into the interface, and improves the connection stability, and thus improves the yield rate of qualified products.
7. In the present disclosure, the first adhesive layer and/or the soft sealing ring and/or the sealing block are used to block possible gaps (openings) on the first (second) shell part, thereby realizing a sealed and closed space, and preventing the external environment from damaging the electronic components inside the recorder. Thereby, it facilitates the assembly process and improving the yield rate of qualified products.
8. In one embodiment, at least a portion of the connecting end is fixed to the shell (first shell part) by injection molding. To be specific, at first a portion of the connecting end of the flexible patch is inserted into a mold for injection molding, and then the lower shell (first shell part) is formed by injection molding in the mold. In this way, the connecting end is prevented from moving or misaligning during the assembly. Besides, the opening on the bottom of the shell can be fully blocked by injection molding, so as to improve the sealing of the shell.
9. In the fully-disposable ECG recorder provided in the present disclosure, a limiting member may be provided in the shell, to limit the position of the antenna, so that a certain distance is maintained between the antenna and the battery. Thereby, it improves the radiation performance of the antenna, reduces interference/distortion to signals, and improves the yield rate of qualified products.
10. In the fully-disposable ECG recorder provided in the present disclosure, an insulating pull tab is provided and can be pulled out during use. Before being pulled out, the insulating pull tab plays the role of electrically isolating the battery from the signal processing unit, so that the fully-disposable ECG recorder remains in a turned-off state when not in use. During use, the ECG recorder can be quickly turned on by a user pulling out the insulating pull tab.
11. The flexible patch may comprise an adhesive patch layer and an electric layer. From top to bottom, the electric layer comprise a substrate layer, an electrical path layer, an isolation layer, and a sticky layer. Therefore, a complex structure is avoided, and a foam layer is avoided. Thus, the flexible patch is light and soft, and has good fit and comfort when being worn.

### Description of the drawings

In order to more clearly illustrate the detailed implementation of the present disclosure or the technical solution in the prior art, the drawings required for illustrating the detailed implementation or the prior art will be briefly introduced below.
Figure 1 is a schematic diagram of the structure of a fully-disposable ECG recorder according to an embodiment of the present disclosure.
Figure 2 is an exploded view of a fully-disposable ECG recorder according to an embodiment of the present disclosure.
Figure 3 is a schematic diagram of the structure of the electric layer according to an embodiment of the present disclosure.
Figure 4 is a cross-sectional view along the line HH' in Figure 3.
Figure 5 is a schematic diagram of the flexible patch at a lower side (the side facing the skin) according to an embodiment of the present disclosure.
Figure 6 is a schematic diagram of the structure inside the shell according to an example of the present disclosure.
Figure 7 is an enlarged view of the area A in Figure 6.
Figure 8 is a schematic diagram of the first shell part according to an embodiment of the present disclosure.
Figure 9 is a schematic diagram of the second shell part according to an embodiment of the present disclosure.
Figure 10 is a schematic diagram of the structure of a fully-disposable ECG recorder according to another example of the present disclosure.
Figure 11 is a schematic diagram of the first shell part according to the example of Figure 10.
Figure 12 is a schematic diagram of the structure inside the shell according to an embodiment of the present disclosure.
Figure 13 is a schematic diagram of the structure in which the connecting end extends into the interior of the shell according to an embodiment of the present disclosure.

### Description of reference signs:

1 - flexible patch, 11 - adhesive patch layer, 11a - lower surface (surface facing toward the skin), 11b - upper surface (surface facing away from the skin), 11c - second opening, 12 - electric layer, 121 - electrode, 122 - flexible electrical path, 122a - connecting end, 122b - substrate layer, 122c - electrical path layer, 122d - isolation layer, 122e - sticky layer, 122f - reinforcement sheet, 123 - conductive gel, 13 - first adhesive layer, 14 - second adhesive layer, 14a - third opening, 15 - lower release film, 16 - upper release film; 2 - shell, 21 - first shell part, 210 - fixing member, 211 - accommodating structure, 211a - claw, 212 - positioning structure, 212a - first positioning wall, 212b - second positioning wall, 212c - support rib, 212d - receiving slot, 213 - strip-shaped groove, 214 - receiving part, 215 - soft sealing ring, 216 - first opening, 217 - limiting member, 218 - through hole, 219 - recess, 22 - second shell part, 221 - mating part, 3 - signal processing unit, 31 - circuit board, 311 - interface, 312 - connecting member, 312a - first contact, 312b - second contact, 313 - spring connector, 32 - chip, 34 - antenna, 4 - insulating pull tab, 5 - battery, 5a - first electrode, 5b - second electrode, 5c - first connector, 5d - second connector, 5e - spring sheet.

### Detailed Embodiments

As below, the technical solution of the present disclosure will be described clearly and completely in conjunction with the accompanying drawings. Evidently, the described embodiments are only a part of the embodiments of the present disclosure, rather than all of the embodiments.

In the description of the present disclosure it should be noted that the terms indicating the orientation or position relationship, such as "center", "upper", "lower", "left", "right", "vertical", "horizontal", "inner", "outer", etc., are based on the orientation or position relationship shown in the drawings. And these terms are only used for the convenience of describing the present disclosure, and do not intend to indicate or imply that the device or element must have such a specific orientation, or must be constructed/operated in such a specific orientation. Therefore, these terms should not be understood as a limitation on the present disclosure. In addition, the terms such as "first" and "second" are used only for descriptive purposes, and cannot be understood as indicating or implying any relative importance.

Besides, the technical features involved in different embodiments of the present disclosure may be combined with one another, as long as they do not conflict with each other.

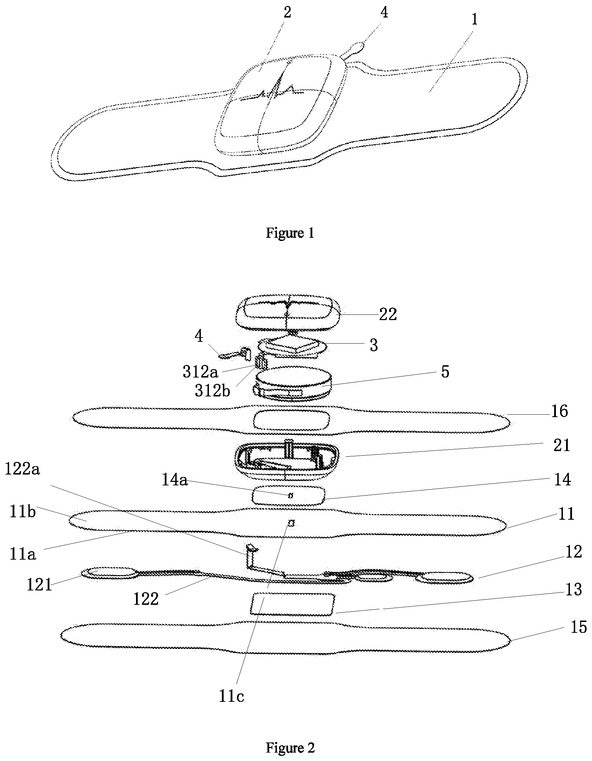

As shown in Figures 1 to 3, the present invention provides a fully-disposable ECG recorder, which comprises a flexible patch 1 and a shell 2. The flexible patch 1 comprises an electric layer 12, and the electric layer 12 comprises at least two electrodes 121 and a flexible electrical path 122 electrically connected to the electrodes 121. The flexible patch 1 is configured to be attached to the surface of a human body. The electrodes 121 are able to acquire/collect ECG signals. The signals are transmit via the flexible electrical path 122.

The shell 2 comprises a first shell part (lower shell) 21 and a second shell part (upper shell) 22. The first shell part 21 and the second shell part 22 are fixedly (non-detachably) connected together, so as to form an internal space. The lower surface of the first shell part 21 is fixedly (non-detachably) connected to the flexible patch 1, so as to form an integrated ECG recorder. During use, the ECG recorder does not need assembly, and after use, the ECG recorder can be completely discarded .

The shell 2 has a closed internal space. The internal space is enclosed by the shell 2 and thus is limited by the shell 2. The closed space is in a substantially sealed state, so that the internal components, such as the signal processing unit 3, can be protected from corrosion by water vapor, sweat, etc.. The signal processing unit 3 is used to process the acquired ECG signals, so as to obtain ECG data. The processing may include operations such as filtering, amplification, AD conversion, etc.

The flexible electrical path 122 has a connecting end 122a, which extends upward from the flexible patch 1 into the closed space of the shell 2. The connecting end 122a is connected to the signal processing unit 3.

The fully-disposable ECG recorder provided by the present invention has a shell 2 and a flexible patch 1 which are fixed together. During use, assembling or disassembling are not needed; after use, the ECG recorder can be completely discarded. Thereby, the user convenience can be improved.

Besides, the connecting end of the flexible electrical path extends from the outside of the shell into the closed space inside the shell, and is connected to the signal processing unit in the closed space. Thereby, it can overcome the problems in the prior art, which are caused by the connection position being arranged outside the shell, such as poor connection stability, susceptibility to external environmental influences such as temperature and humidity.

Furthermore, in the ECG recorder provided in the present disclosure, the signal processing unit is accommodated in a closed shell, and the sealing of the signal processing unit and other electronic components can be achieved without using a process of heat laminating or heat sealing. Unlike heat laminating/sealing, the manufacturing method of the present disclosure will not cause damage to the electronic elements inside the recorder. The manufacturing process is simple and the yield rate of qualified products is high.

In the present invention, the number of the electrodes 121 may not be limited. The number of the electrodes 121 may be 2 or more, such as 3, 4, 5, 6, 7 or 8.

In the present disclosure, the structure/composition of the signal processing unit 3 may not be limited. Optionally, the signal processing unit 3 may comprise a circuit board 31 and a chip 32. The chip 32 may be fixed on the circuit board 31. Optionally, the chip 32 comprises a memory and a processor. The memory is configured to store the ECG signals acquired by the electrodes, and the processor is configured to process the ECG signals acquired by the electrodes, so as to obtain ECG data.

Referring to Figure 2, optionally, the flexible patch 1 comprises an adhesive patch layer 11, which has a lower surface (surface facing toward the skin) 11a and an upper surface (surface facing away from the skin) 11b. The shell 2 is fixed on the upper surface 11b, and the electric layer 12 is fixed on (e.g adheres to) the lower surface 11a. At least a portion of the flexible electrical path 122 is spread on the lower surface 11a, and is connected to each electrode 121.

The flexible patch 1 provided by the present invention may only consist of an adhesive patch layer 11 and an electric layer 12. By avoiding a complex multi-layer structure, the flexible patch 1 can have a simple structure and a light weight, which improves the wearer's comfort. In the layered structure, the flexible patch of the present disclosure does not have a foam layer, so the flexible patch can be light and soft, and it can fit the human body better when being worn.

Optionally, the adhesive patch layer 11 may be a single-sided tape, especially a medical single-sided tape. As a single-sided tape, the lower surface 11a is sticky, while the upper surface 11b is not sticky.

In the present disclosure, the relative position between the shell 2 and the adhesive patch layer 11 may not be limited. Optionally, the shell 2 may be located in the middle area of the upper surface 11b.

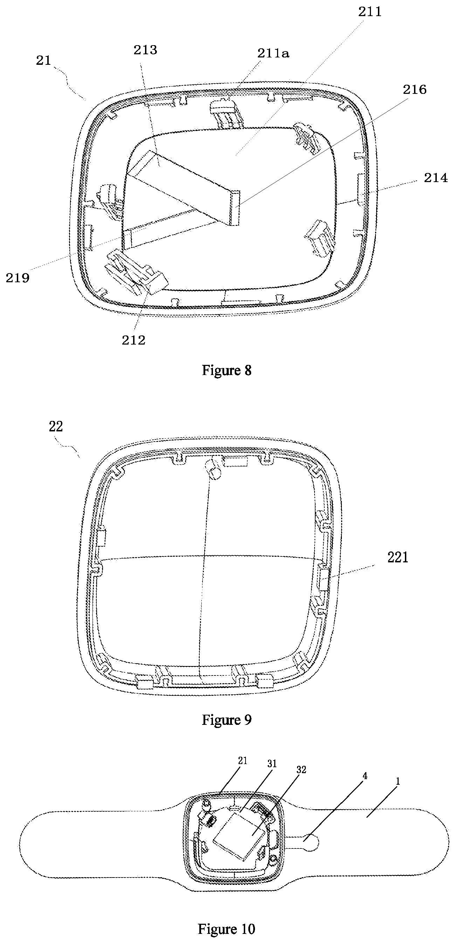

Referring to Figures 2 and 8, the lower surface of the first shell part 21 has a first opening 216. More preferably, the adhesive patch layer 11 has a second opening 11c. The connecting end 122a of the flexible electrical path 122 extends upward and passes through the first opening 216 (and the second opening 11c) into the closed space inside the shell 2. In a specific embodiment, the first opening 11c is located in the middle area of the adhesive patch layer 11; the second opening 216 is located in the middle area of the first shell part 21.

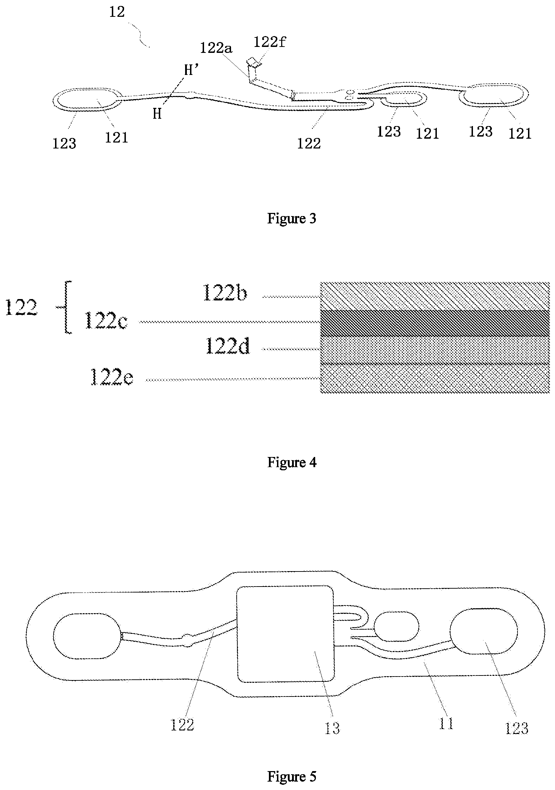

Referring to Figure 5, optionally, the flexible patch 1 is further provided with a first adhesive layer (lower adhesive layer) 13. The first adhesive layer (lower adhesive layer) 13 is disposed below the electric layer 12. The first adhesive layer 13 is used to cover the first opening 216 of the shell 2 (and the second opening 11c of the adhesive patch layer 11), so that the space inside the shell 2 is a closed space. Optionally, the first adhesive layer 13 covers at least a portion of the flexible electrical path 122, but does not cover any electrode 121. When the flexible patch 1 is attached to the human body, the first adhesive layer 13 can prevent human secretions, such as sweat and other substances, from entering the interior of the shell 2 through the first opening 216, thereby improving the reliability of the signal processing unit 3 in the shell 2.

Further referring to Figure 2, optionally, the flexible patch 1 is further provided with a second adhesive layer (upper adhesive layer) 14. For example, the second adhesive layer 14 is used to adhere to the shell 2, so that the lower surface of the shell 2 is fixedly connected to the flexible patch 1. For example, the second adhesive layer 14 is located between the shell 2 and the adhesive patch layer 11, and is used to fix the shell 2 to the upper surface 11b of the adhesive patch layer 11. Optionally, the second adhesive layer 14 may be a double-sided adhesive layer. As a double-sided adhesive layer, the second adhesive layer 14 is used to fix the first shell part 21 to the adhesive patch layer 11 together.

In some specific embodiments, the second adhesive layer 14 has a third opening 14a. The connecting end 122a of the flexible electrical path 122 passes through the first opening 11c of the adhesive patch layer 11, the third opening 14a of the second adhesive layer 14, and the second opening 126 of the shell 2, so as to enter the closed space inside the shell.

Figure 3 is a schematic diagram of an electric layer provided in an embodiment of the present disclosure, and Figure 4 is a schematic cross-sectional view along the line HH' in Figure 3. As shown in the figures, in some embodiments, the lower side of the electric layer 12 is provided with a sticky layer 122e. The upper side of the sticky layer 122e is fixed to the flexible electrical path 122. When the ECG recorder is attached to the human body, the lower side of the sticky layer 122e is in contact with the human body. In this embodiment, the sticky layer 122e covers at least a portion of the flexible electrical path 122, but does not cover any electrode 121. The sticky layer 122e can increase the adhesion to skin, which helps to place/spread the electric layer 12 on the surface of human skin. Due to the existence of the sticky layer 122e, the electrodes 121 can be more stably attached to the surface of human skin, thereby enhancing the reliability of ECG signal acquisition.

Optionally, the electric layer 12 is further provided with an isolation layer 122d. The isolation layer 122d may be located under the flexible electrical path 122. The isolation layer 122d may be located above/on the sticky layer 122e. For example, The isolation layer 122d is located between the sticky layer 122e and the flexible electrical path 122. The isolation layer 122d is used to isolate the flexible electrical path from the human body. Optionally, the isolation layer 122d can be made from electrically insulating ink. The isolation layer 122d can prevent human secretions, such as sweat, from entering into the flexible electrical path 122.

In the present disclosure, the composition/structure of the flexible electrical path 122 may not be limited. Optionally, the flexible electrical path 122 comprises a substrate layer 122b and an electrical path layer 122c. During manufacturing, the electrical path layer 122c may be applied (printed) on the substrate layer 122b. In a specific embodiment, the material of the electrical path layer 122c is copper.

Therefore, the electric layer 12 may have a substrate layer 122b, an electrical path layer 122c, an isolation layer 122d, and a sticky layer 122e from top to bottom. In one embodiment, the electric layer 12 (non-electrode portion) of the present disclosure consists of four layers: a substrate layer 122b, an electrical path layer 122c, an isolation layer 122d, and a sticky layer 122e. Since the flexible patch 1 of the present disclosure does not comprise a foam layer, the flexible patch 1 is light and soft and comfortable to wear.

In practices, the electrode 121 may comprise a silver layer and/or a silver chloride layer. For example, a silver chloride layer may be placed over/on the silver layer. At least one selected from the silver layer and the silver chloride layer is connected to the electrical path layer 122c of the flexible electrical path 122. The existence of the silver chloride layer can increase the effectiveness of signal conduction. The silver layer may be formed by conductive silver paste.

Further referring to Figure 3, conductive gel 123 may be provided below/under the electrode(s) 121. When in use, the conductive gel 123 is in contact with human skin, which helps to improve conductivity and to reduce the impedance between the skin and the electrode.

Further referring to Figure 2, optionally, an upper release film 16 and a lower release film 15 are respectively provided on the upper and lower sides of the flexible patch 1. For example, the upper release film 16 and the lower release film 15 cover the upper and lower sides of the adhesive patch layer 11, respectively. The lower release film 15 also covers the electric layer 12 being fixed on the lower side of the adhesive patch layer 11, so as to protect the adhesive patch layer 11 and the electric layer 12. When in use, the upper release film 16 and the lower release film 15 can be peeled off.

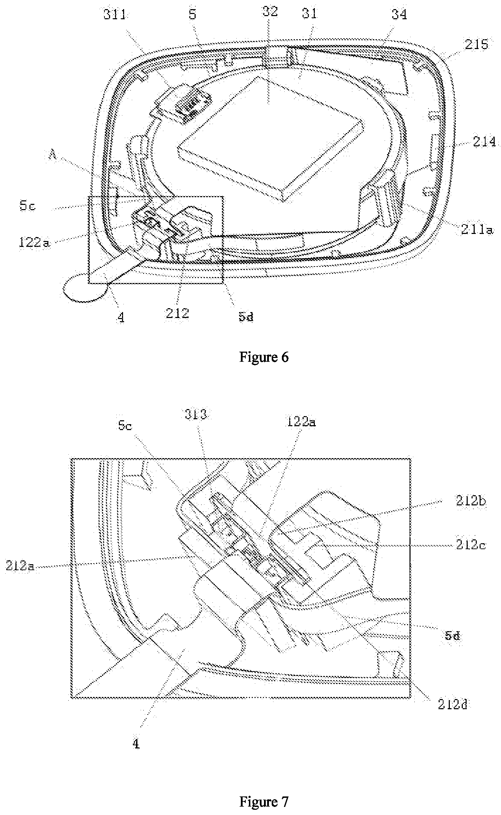

Referring to Figures 6-9, optionally, the second shell part 22 and the first shell part 21 are fixed in a non-detachable manner. It should be understood that the "being fixed in a non-detachable manner" in our invention means that the two parts cannot be separated during normal use, that is, they cannot be easily separated without the aid of tools, they cannot be non-destructively separated, etc.

For example, the second shell part 22 and the first shell part 21 are connected by snap-fit joint, especially inseparable snap-fit joint. For example, multiple receiving parts 214 may be provided on/near the edge (preferably on the inner side) of one shell part (the second shell part 22 and/or the first shell part 21), and multiple mating parts 221 (such as snap-fitting hooks) (e.g. having undercut(s)) corresponding to the receiving parts 214 are provided (preferably on the inner side) in the other shell part (the first shell part 21 or/and the second shell part 22). When the receiving parts 214 and the mating parts 221 are engaged one by one, the second shell part 22 and the first shell part 21 are connected, preferably in an inseparable manner.

Optionally, a soft sealing ring 215 may be provided at the mating surface between the second shell part 22 and the first shell part 21. Preferably, the soft sealing ring 215 is not visible from outside the shell, so as to improve the sealing effect of the shell. The soft sealing ring 215 may be made from soft rubber / soft plastics / soft silicone, etc.. In a specific embodiment, the soft sealing ring 215 and the first shell part 21 (the second shell part 22) may be formed by two-shot injection molding.

As an alternative to snap-fit joint, the second shell part 22 and the first shell part 21 of the fully-disposable ECG recorder may be fixed together by ultrasonic welding, or by glue bonding, such as bonding with glue spots.

Referring to Figure 6, optionally, an interface 311 is provided on the circuit board 31. The interface 311 is to be connected to the connecting end 122a of the flexible electrical path 122. Through the interface 311, the electrical connection between the flexible electrical path 122 and the signal processing unit 3 is achieved. To be specific, the interface 311 may be a ZIF interface, and the connecting end 122a of the flexible electrical path 122 may be a ZIF terminal. The connection achieved by the ZIF interface and the ZIF terminal has the advantages of high reliability, increased ease of use, and longer service life.

Optionally, in order to facilitate the inserting the connecting end 122a of the flexible electrical path 122 into the interface 311, and in order to to improve the connection stability, a reinforcing sheet 122f may be provided on the connecting end 122a.

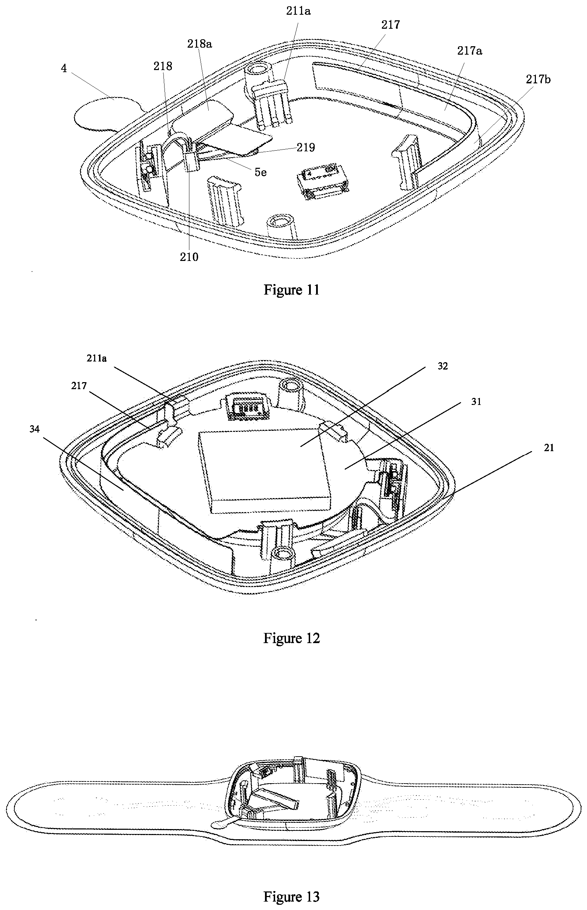

As shown in Figure 3 and Figure 13, the reinforcing sheet 112 f is provided at one end of the connecting end 122a. The reinforcing sheet 112f usually has a stronger Young's modulus than the main body of the flexible electrical path 122. The existence of the reinforcing sheet 112f facilitates the assembly process during manufacture, which makes it easier to insert the connecting end 122a into the interface 311 .

In a specific embodiment, an end of the reinforcing sheet 122f may be bent upward relative to the connecting end 122a, thereby forming a handle for inserting.

As an alternative, the reinforcing sheet 122f may not be bent, but only extend backwards, which can also form a handle for inserting, and/or for stabilizing the connection.

Referring to Figures 2, 6 and 8, an accommodating structure 211 is provided in the first shell part 21. The accommodating structure 211 is used for accommodating battery 5 and/or the circuit board 31. For example, the battery 5 is accommodated (such as clamped) in the accommodating structure 211, and the circuit board 31 is placed (stacked) over/on the battery 5.

In the present disclosure, the structure/composition of the accommodating structure 211 may not be limited. Optionally, the accommodating structure 211 may comprise multiple claws 211a. The claws 211a may be arranged to surround the battery 5, so as to facilitate the assembly and fixation of the battery in the shell. The claws 211a may be configured to clamp/hold the battery 5 and/or the circuit board 31. Optionally, the circuit board 31 may be assembled over the battery 5 by adhering/gluing, or the circuit board 31 may be clamped over the battery 5 by the claws 211a of the accommodating structure 211.

Optionally, after the connecting end 122a of the flexible electrical path 122 enters the space inside the shell 2 (inside the lower shell part 21), it is bent multiple times and then inserted into the interface 311 .

As shown in Figure 13, during assembly, the connecting end 122a of the flexible electrical path 122 enters the first shell part 21, and at first extends horizontally under the bottom of battery 5, and then extends upwards at the edge of the battery 5 (or at the edge of the circuit board 31), and then is inserted into the interface 311. That is, the portion of the connecting end 122a entering the first shell part 21 is bent for a first time for extending parallel to the bottom surface of the battery 5, and then is bent for the second time at the edge of the battery 5 (or at the edge of the circuit board 31) for extending upward, and then is bent for the third time toward the interface 311 (e.g. at the top/edge of the circuit board 31) for being inserted into the interface 311. This kind of design ensures that the flexible electrical path 122 does not occupy any extra space in the shell 2, which is conducive to miniaturization of the device.

When the edge of the circuit board 31 is more outward than the edge of the battery 5, the portion of the connecting end 122a of the flexible electrical path 122 entering the first shell part 21 may be bent for a first time for extending parallel to the bottom surface of the battery 5, and then is bent for a second time at the edge of the circuit board 31 for extending upward, and then is bent for a third time toward the interface 311 (e.g. at the top/edge of the circuit board 31). When the edge of the circuit board 31 is located close to the edge of the battery 5, the portion of the connecting end 122a of the flexible electrical path 122 entering the first shell part 21 is bent for a first time for extending parallel to the bottom surface of the battery 5, and then is bent for a second time at the edge of the battery 5 and/or the circuit board 31 for extending upward, and then is bent for a third time toward the interface 311 (e.g. at the top/edge of the circuit board 31).

Optionally, a bending segment or multiple bending segments may be provided when the connecting end 122a is bent for the second/third time. The existence of multiple bending segments can facilitate adjusting the lateral distance between the connecting end 122a and the interface 311 during the assembly process, so as to improve the fault tolerance in the assembly process.

Optionally, as shown in Figure 8, the first shell part 21 is provided with a strip-shaped groove 213. The portion of the connecting end 122a extending parallel to the bottom surface of the battery 5 may be received/accommodated in the strip-shaped groove 213. Thus, the connecting end 122a does not occupy any extra space in the shell.

Optionally, at least a portion of the connecting end 122a is fixed to the first shell part 21 by injection molding. To be specific, at first a portion of the connecting end 122a of the flexible patch 1 is inserted into a mold for injection molding, and then the lower shell (first shell part) 21 is formed by injection molding in the mold. For example, the portion of the connecting end 122a extending parallel to the bottom surface of the battery 5 and the portion of the connecting end 122a passing through the first shell part 21 are fixed to (integrated with) the first shell part 21 by injection molding. In this way, the position of the connecting end 122a can be fixed by injection molding, so as to prevent the connecting end 122a from being moved or misaligned during the assembly process. Besides, the opening on the bottom of the shell can be blocked by injection molding, so as to improve the sealing of the shell.

Thereby, by the injection molding with a portion of the connecting end 122a in the mold, the lower shell (first shell part) 21 is formed and obtained with the connecting end 122a passing through the the lower shell 21 and entering the space inside the lower shell 21.

In this embodiment, the connecting end 122a of the flexible patch 1 may first pass through the third opening 14a of the second adhesive layer 14, and then be inserted into the mold for injection molding.

Referring to Figure 11 and Figure 12, the signal processing unit 3 may further comprise an antenna 34. The antenna 34 is configured to send the ECG data from the signal processing unit 3 to an external device (such as a server or a client terminal). Optionally, the antenna 34 may be integrated with the circuit board 31. That is, the antenna 34 and the circuit board 31 are formed/produced at the same time. To be specific, as shown in Figure 12, the antenna 34 extends from an edge side of the circuit board 31 and to a gap between the battery 5 and the first shell part 21. Optionally, the antenna 34 may has a shape of a sheet.

Optionally, as shown in Figures 11 and 12, a limiting member 217 is further provided in the shell 2 (e.g., in the lower shell 21). The limiting member 217 is used to limit the position of the antenna 34, so that the antenna 34 does not contact the battery 5. Because the shell of the battery 5 is made of metal, it will harm the signal transmission if the antenna 34 is in contact with or too close to the battery 5. The existence of the limiting member 217 can facilitate the assembly and improve the yield rate of qualified products.

Further referring to Figure 11, the limiting member 217 forms a strip-shaped protrusion in the first shell part 21. The limiting member 217 (strip-shaped protrusion) is located along the edge(s) of the battery. For example, the strip-shaped protrusion comprises a first surface 217a facing the battery 5 and a second surface 217b opposite to the first surface 217a. The antenna 34 is assembled on (e.g. attached to) the second surface 217b. For example, the antenna 34 may be in contact with the second surface 217b. Optionally, the assembly may be achieved with adhesive or without adhesive. When without adhesive, during assembly the antenna 34 may be simply placed on the side of the second surface 217b of the limiting member 217.

Optionally, the limiting member 217 has a length which is greater than or equal to the length of the antenna 34. In this way, the reliability of limiting the position of antenna 34 can be guaranteed. Because the antenna 34 can be made of flexible material, it is easy to bend. If the length of the limiting member 217 is less than the length of the antenna 34, it is possible that the tail of the antenna 34 may contact the battery. By ensuring the length of the limiting member 217 greater than or equal to the length of antenna 34, this problem can be eliminated.

In the present disclosure, the material of the limiting member 217 may not be limited. The material of the limiting member 217 may be plastic or other materials. Preferably, the limiting member 217 and the first shell part (lower shell) 21 are integrally formed. For example, they may be integrally formed by injection molding. Of course, it is not limited thereto. The limiting member 217 and the first shell part 21 may also be formed separately and then assembled together. For example, during assembly, they are fixed by glue.

As an alternative, the antenna 34 may also be assembled on/over the upper surface of the limiting member 217. In this case, the antenna is not only kept away from the battery, but also be kept away from the human body, so as to decrease the impact of electrical radiation on the human body.

In a more specific embodiment, the upper surface of the limiting member 217 is provided with a groove. The groove is configured to accommodate at least a part of the antenna 34. That is, after assembly, at least a part of the antenna 34 is located in the groove. The groove can strongly limit the position of the antenna 34, which makes the assembly process simpler and more reliable.

In the present disclosure, the shape, number and/or position of the limiting member 217 may not be limited. Besides the strip-shaped protrusion shown in Figure 11, the limiting member 217 may also be elliptical or other shapes. Regarding the number, the number of limiting member(s) may be one or two or more. For example, two or more limiting members are discretely distributed in the shell to jointly limit the position of the antenna.

As shown in Figure 11, the limiting member 217 may be provided in the first shell part 21. However, it is not limited thereto. The limiting member 217 may be provided in the second shell part 22. When the first shell part 21 is connected to the second shell part 22, the limiting member 217 can limit the position of the antenna 34, so as to keep the antenna 34 being away from the battery 5. For example, when the first shell part 21 is connected to the second shell part 22, the limiting member 217 is located between the antenna 34 and the battery 5.

Referring to Figures 2, 6-7, 10-11, optionally, the fully-disposable ECG recorder further comprises an insulating pull tab 4. The insulating pull tab 4 extends from the inside of the shell 2 to the outside of the shell 2. The insulating pull tab 4 is removable, i.e. can be pulled out. Before being pulled out, the insulating pull tab is configured to interrupt/break/cut off the electrical connection, for example the electrical connection to/in the signal processing unit 3. For example, before being pulled out, the insulating pull tab 4 is configured to electrically isolate the battery 5 from the signal processing unit 3.

Optionally, the battery 5 comprises a first electrode 5a and a second electrode 5b. The signal processing unit 3 comprises a connecting member 312. The connecting member 312 comprises a first contact 312a and a second contact 312b. The first electrode 5a and the second electrode 5b are electrically connected to the first contact 312a and the second contact 312b through a first connector 5c and a second connector 5d, respectively. Optionally, the battery 5 may be a button battery. The first electrode 5a may be a positive electrode or a negative electrode of the battery 5, and the second electrode 5b may be the other electrode (a negative electrode or a positive electrode) of the battery 5.

In the present disclosure, the position of the insulating pull tab 4 may not be limited. It is sufficient, as long as the position of the insulating pull tab 4 can cut off the electrical connection, especially the electrical connection between the battery 5 and the signal processing unit 3. For example, the insulating pull tab 4 may be located between the first electrode 5a and the first connector 5c, or the insulating pull tab 4 may be located between the second electrode 5b and the second connector 5d, or the insulating pull tab 4 may be located between the first connector 5c and the first contact 312a, or the insulating pull tab 4 may be located between the second connector 5d and the second contact 312b.

As an example, the arrangement of the insulating pull tab 4 is shown in Figures 6 and 7. The insulating pull tab 4 may extend from the inside of the shell 2 to the outside of the shell 2, passing through the mating surface between the second shell part 22 and the first shell part 21. In this embodiment, this arrangement of the insulating pull tab 4 has the advantages of convenience, simplicity, ect., which can simplify the assembly process of the ECG recorder. Besides, a soft sealing ring 215 may be arranged at the mating surface of the first shell part 21 and the second shell part 22. After assembly, the soft sealing ring 215 is configured to squeeze/press the insulating pull tab 4, so as to guarantee the sealing of the shell 2.

As another example, the arrangement of the insulating pull tab 4 is shown in Figure 11. A through hole 218 is provided near/on the edge of the first shell part 21. The insulating pull tab 4 extends from the inside of the shell 2 to the outside of the shell 2, through the through hole 218. In a more specific embodiment, a sealing block 218a is further provided and is used to block the through hole 218, so as to form a closed space in the shell 2.

Besides, preferably, an electrical spring contact may be used at the position where the electrical connection is cut off by the insulating pull tab, so as to achieve a successful electrical connection after the insulating pull tab is removed (pulled out). The use of electrical spring contacts can improve the yield rate of the qualified products.

An example of the electrical connection with spring contact(s) is shown in Figure 11, and another example is shown in Figures 6-8 .

In the example of Figure 11 , the first connector 5c and/or the second connector 5d has a spring sheet 5e. In an example, the first electrode 5a is located on the bottom surface of the battery 5, the spring sheet 5e is located between the battery 5 and the first shell part 21. The insulating pull tab 4 is located between the first electrode 5a and the spring sheet 5e. The spring sheet 5e abuts against the bottom surface of the insulating pull tab 4. After the insulating pull tab 4 is pulled out, the spring sheet 5e abuts against the bottom surface of the first electrode 5a. In this example, the first electrode 5a is located on the bottom surface of the battery 5, the insulating pull tab 4 electrically isolates the first electrode 5a from the spring sheet 5e. The spring sheet 5e has a resilient force, which causes the end of the spring sheet 5e to apply an upward abutting force on the bottom surface of the insulating pull tab 4. Preferably, the end of the spring sheet 5e is provided with protrusion(s). The existence of the protrusion(s) can further improve the connection reliability between the spring sheet 5e and the first electrode 5a.

Further referring to Figure 11, a recess 219 is provided on the inner surface of the first shell part 21, so as to accommodate at least a part of the first connector 5c and/or the second connector 5d. For example, the shape of the recess 219 matches the shape of the spring sheet 5e. At least a part of the spring sheet 5e contacts the surface of the recess 219. The recess 219 is provided to accommodate at least a part of the spring sheet 5e of the first connector 5c and/or the second connector 5d. It ensures that the battery 5 can be flatly assembled in the first shell part 21. Optionally, the depth of the recess 219 is between 0.1 mm and 0.5 mm, for example, 0.2 mm.

Optionally, referring to Figure 11, in order to facilitate fixing the first connector 5c in the second shell 21, a fixing member 210 is provided in the first shell part 21. The fixing member 210 may be provided adjacent to the recess 219. At least a part of the first connector 5c is fixed in the fixing member 210. The fixing member 210 is configured to limit the position of the first connector 5c (so as to limit the position of the spring sheet 5e). It can be understood that the presence of the fixing member 210 can fix the position of the first connector 5c, so that the position of the first connector 5c will not change with the shaking of the shell 2. More importantly, the fixing member 210 can enable the spring sheet 5e to stably abut against the first electrode 5a, thereby improving the reliability of the electrical connection.

In another example (such as Figures 6-8), a spring connector 313 is provided to achieve electrical connection between the first connector 5c and the first contact 312a (and/or between the second connector 5b and the second contact 312b).

Referring to Figures 6 and 7, Figure 6 is a schematic diagram of the internal structure of the shell, and Figure 7 is an enlarged view of the area A in Figure 6.

As shown in the figures, a positioning structure 212 is arranged in the first shell part 21, and the positioning structure 212 comprises a first positioning wall 212a and a second positioning wall 212b which are arranged opposite to each other. For example, an end of the first connector 5c and/or an end of the second connector 5b are arranged on the inner side of the first positioning wall 212a, and the connecting part 312 of the circuit board 31 is arranged on the inner side of the second positioning wall 212b. That is, the connecting part 312 of the circuit board 31 is located opposite the end of the first connector 5c and/or the end of the second connector 5b.

A first spring connector 313 is located between the end of the first connector 5 and the first contact 312a of the connector 312, and/or a second spring connector 313 is located between the end of the second connector 5b and the second contact 312b of the connector 312.

The positioning structure 212 and the spring connector 313 are combined and cooperate together, to achieve a stable connection between the circuit board 31 and the battery 5. Compared with the welding connection in prior art, this kind of connection in the disclosure is more reliable and will not cause any high-temperature damage to the circuit board 31.

As shown in Figure 7, the first positioning wall 212a may be close to the battery 5. Optionally, a support rib 212c may be provided at the outer side of the first positioning wall 212a. The support rib 212c preferably conforms to the outer periphery of the battery 5. In the positioning structure 212, the support rib 212c can play a role in assisting the support of the battery 5. For example, under the impact of external force (for example, when the ECG recorder falls, etc.), the relative position of the battery 5 can be kept stable.

A receiving slot 212d is provided on the inner side of the first positioning wall 212a, and faces towards the second positioning wall 212b. During assembly, the connecting member 312 of the circuit board 31 can be inserted/accommodated in the receiving slot 212d. The first connector 5c and the second connector 5d extend from two different sides to the gap between the first positioning wall 212a and the second positioning wall 212b.

Optionally, in the fully-disposable ECG recorder of the present disclosure, the shell 2 has a structure like a flat box, whose horizontal cross-section is a rectangle or square, preferably a rectangle/square with curved corners and/or curved edges. More preferably, the upper surface of the shell 2 has a continuously curved surface.

Optionally, in the fully-disposable ECG recorder provided by the present disclosure, the positioning structure 212, the interface 311, and the antenna 34 are placed at/near different corners in the shell 2, so that the whole layout can make full use of the space inside the shell 2, which is conducive to miniaturization of the device.

In some embodiments, a process of assembling/manufacturing the fully-disposable ECG recorder of the present disclosure may be described as follows.

Step 1, forming a flexible patch 1, and leading the connecting end 122a out for extending/bending upward. For example, after leading the connecting end 122a of the flexible electrical path 122 to pass through the second opening 11c of the adhesive patch layer 11, fixing at least a part of the electric layer 12 on the adhesive side of the adhesive patch layer 11 (by adhering), thereby forming the flexible patch 1.

Step 2, leading the connecting end 122a of the flexible patch 1 to enter the space inside the lower shell (first shell part) 21.

In one embodiment, at first, a lower shell 21 with an first opening 216 on the bottom side is formed, and then the connecting end 122a of the flexible patch 1 passes through the first opening 216 of the lower shell 21 and enters the space inside the lower shell 21. In this embodiment, the connecting end 122a of the flexible patch 1 may first pass through the third opening 14a of the second adhesive layer 14 and then pass through the first opening 216 of the lower shell 21.

In another embodiment, at first, a portion of the connecting end 122a of the flexible patch 1 is inserted into a mold for injection molding, and then the lower shell (first shell part) 21 is formed by injection molding in the mold. Thereby, the connecting end 122a of the flexible patch 1 passes through the lower shell (first shell part) 21 and enters the space inside the lower shell 21. In this embodiment, the connecting end 122a of the flexible patch 1 may first pass through the third opening 14a of the second adhesive layer 14 and then be inserted into the mold for injection molding.

Step 3, installing the signal processing unit 3 in the space inside the lower shell 21. For example, firstly installing the battery 5, then installing the circuit board 31 (including installing the antenna 34, etc.), and finally inserting the connecting end 122a of the flexible patch 1 into the interface 311 of the circuit board 31.

Step 4, optionally, installing the insulation pull tab 4.

Step 5, installing the second shell (upper shell) 22, for example covering the upper shell 22 over the lower shell 21, and fixing the first shell 21 and the first shell 22 together, so that the first shell 21 and the first shell 22 are fixedly (non-detachably) connected.

Step 6, other installations. For example, optionally, the first adhesive layer 13 is applied on the lower side of the flexible patch 1, so as to cover the opening(s). For example, the upper release film 15 and the lower release film 16 are laid on the upper and lower sides of the flexible patch 1, respectively.

The present disclosure provides an ECG monitoring system, which comprises a fully-disposable ECG recorder as described in the above embodiments, as well as a client terminal and/or a server. The client terminal may be an application on a mobile device. The mobile device may be, for example, a mobile phone, a computer, a tablet, etc. The client terminal may be configured to receive the ECG data sent by the fully-disposable ECG recorder, and configured to store and/or to draw and/or to display the data to the user. Optionally, the client terminal and/or the ECG recorder is also configured to communicate with the server and is configured to send the ECG data to the server. After receiving the ECG data, the server may be configured to process the ECG data. The processing can include, e.g. storing, analyzing, etc.

## Claims

1. A fully-disposable electrocardiograph, ECG, recorder, comprising
a flexible patch (1), configured to be attached to the surface of a human body, the flexible patch comprises an electric layer (12), the electric layer comprises a flexible electrical path (122) and at least two ECG electrodes (121), wherein the flexible electrical path is connected to the at least two ECG electrodes;
a shell (2), forming a closed space;
a signal processing unit (3),
wherein the signal processing unit is located in the closed space;
the flexible electrical path has a connecting end (122a),
wherein
the flexible patch is fixed on a bottom skin-facing side of the shell, wherein
the bottom side has a first opening (216) for the connecting end to pass through; and
the connecting end of the flexible electrical path extends upward into the closed space inside the shell, so as to be connected to the signal processing unit.

2. The fully-disposable ECG recorder according to claim 1, wherein
the flexible patch is provided with an adhesive patch layer (11), and the adhesive patch layer has a second opening (11c) corresponding to the first opening; the adhesive patch layer has a lower surface (11a) and an upper surface (11b), a part of the flexible electrical path is spread on the lower surface, and another part of the flexible electrical path is bent so as to enter the closed space through the second opening and the first opening.

3. The fully-disposable ECG recorder according to any of claims 1 to 2, wherein the shell comprises a first shell part (21) and a second shell part (22).

4. The fully-disposable ECG recorder according to claim 3, wherein
the first shell part and the second shell part are separate shell parts, which are non-detachably connected with each other so as to form the closed space.

5. The fully-disposable ECG recorder according to any of claims 1 to 4, wherein a reinforcing sheet (122f) is provided at the connecting end of the flexible electrical path.

6. The fully-disposable ECG recorder according to any of claims 1 to 5, wherein the flexible patch is provided with a first adhesive layer (13), and the first adhesive layer covers the first opening, so as to ensure the closed space in the shell; preferably, the first adhesive layer covers at least a part of the flexible electrical path but does not cover any electrode.

7. The fully-disposable ECG recorder according to any of claims 1 to 6, wherein the flexible patch is provided with a second adhesive layer (14), which is configured to adhere to the shell, so that the lower surface of the shell and the flexible patch are fixedly connected.

8. The fully-disposable ECG recorder according to any of claims 3 to 7 when citing the claim 3, wherein a soft sealing ring (215) is arranged at the matching surface of the second shell part and the first shell part.

9. The fully-disposable ECG recorder according to any of claims 1 to 8, wherein, after the connecting end of the flexible electrical path enters the closed space in the shell, the connecting end is bent multiple times and then connected to the signal processing unit.

10. The fully-disposable ECG recorder according to any of claims 1 to 9, wherein the signal processing unit comprises an antenna (34), a limiting member (217) is provided in the shell, and the limiting member is used to limit the position of the antenna.

11. The fully-disposable ECG recorder according to any of claims 1 to 10, wherein the fully-disposable ECG recorder further comprises an insulating pull tab (4); wherein the insulating pull tab preferably extends from the inside of the shell to the outside of the shell; wherein the insulating pull tab is able to be pulled out; wherein before being pulled out, the insulating pull tab is configured to interrupt or break the electrical connection, for example the electrical connection to or in the signal processing unit.

12. The fully-disposable ECG recorder according to claim 11, wherein electrical connection with a spring contact or contacts is used, and the electrical connection with a spring contact or contacts is cut off by the insulating pull tab.

13. The fully-disposable ECG recorder according to any of claims 1 to 12, wherein from top to bottom, the electric layer has a substrate layer (122b), an electrical path layer (122c), an isolation layer (122d), and a sticky layer (122e).

14. An ECG monitoring system, comprising the ECG recorder according to any one of claims 1 to 13, and a server, configured to receive ECG data from the ECG recorder, wherein the server is configured to process the ECG data after receiving the ECG data from the ECG recorder.

## Patentansprüche

1. Vollständig wegwerfbarer elektrokardiografischer EKG-Recorder, umfassend
ein flexibles Pflaster (1), das dazu konfiguriert ist, an der Oberfläche eines menschlichen Körpers angebracht zu sein, wobei das flexible Pflaster eine elektrische Schicht (12) umfasst, wobei die elektrische Schicht einen flexiblen elektrischen Pfad (122) und zumindest zwei EKG-Elektroden (121) umfasst,
wobei der flexible elektrische Pfad mit den zumindest zwei EKG-Elektroden verbunden ist;
eine Hülle (2), die einen geschlossenen Raum bildet;
eine Signalverarbeitungseinheit (3), wobei sich die Signalverarbeitungseinheit in dem geschlossenen Raum befindet;
der flexible elektrische Pfad ein Verbindungsende (122a) aufweist,
wobei das flexible Pflaster auf einer unteren, der Haut zugewandten Seite der Hülle befestigt ist, wobei
die Unterseite eine erste Öffnung (216) zum Hindurchführen des Verbindungsendes aufweist; und
sich das Verbindungsende des flexiblen elektrischen Pfads nach oben in den geschlossenen Raum innerhalb der Hülle erstreckt, um mit der Signalverarbeitungseinheit verbunden zu sein.

2. Vollständig wegwerfbarer EKG-Recorder nach Anspruch 1, wobei
das flexible Pflaster mit einer Haftpflasterschicht (11) bereitgestellt ist und die Haftpflasterschicht eine zweite Öffnung (11c) entsprechend der ersten Öffnung aufweist; die Haftpflasterschicht eine untere Oberfläche (11a) und eine obere Oberfläche (11b) aufweist,
ein Teil des flexiblen elektrischen Pfads auf der unteren Oberfläche verteilt ist und ein anderer Teil des flexiblen elektrischen Pfads gebogen ist, um in den geschlossenen Raum durch die zweite Öffnung und die erste Öffnung einzutreten.

3. Vollständig wegwerfbarer EKG-Recorder nach einem der Ansprüche 1 bis 2, wobei die Hülle ein erstes Hüllenteil (21) und ein zweites Hüllenteil (22) umfasst.

4. Vollständig wegwerfbarer EKG-Recorder nach Anspruch 3, wobei das erste Hüllenteil und das zweite Hüllenteil separate Hüllenteile sind, die unlösbar miteinander verbunden sind, um den geschlossenen Raum zu bilden.

5. Vollständig wegwerfbarer EKG-Recorder nach einem der Ansprüche 1 bis 4, wobei eine Verstärkungslage (122f) an dem Verbindungsende des flexiblen elektrischen Pfads bereitgestellt ist.

6. Vollständig wegwerfbarer EKG-Recorder nach einem der Ansprüche 1 bis 5, wobei das flexible Pflaster mit einer ersten Haftschicht (13) bereitgestellt ist und die erste Haftschicht die erste Öffnung bedeckt, um den geschlossenen Raum in der Hülle sicherzustellen; bevorzugt die erste Haftschicht zumindest einen Teil des flexiblen elektrischen Pfads bedeckt, aber keine Elektrode bedeckt.

7. Vollständig wegwerfbarer EKG-Recorder nach einem der Ansprüche 1 bis 6, wobei das flexible Pflaster mit einer zweiten Haftschicht (14) bereitgestellt ist, die dazu konfiguriert ist, an der Hülle zu haften, sodass die untere Oberfläche der Hülle und das flexible Pflaster fest verbunden sind.

8. Vollständig wegwerfbarer EKG-Recorder nach einem der Ansprüche 3 bis 7, wenn Anspruch 3 zitiert wird, wobei ein weicher Dichtungsring (215) an der passenden Oberfläche des zweiten Hüllenteils und des ersten Hüllenteils angeordnet ist.

9. Vollständig wegwerfbarer EKG-Recorder nach einem der Ansprüche 1 bis 8, wobei, nachdem das Verbindungsende des flexiblen elektrischen Pfads in den geschlossenen Raum in der Hülle eintritt, das Verbindungsende mehrfach gebogen und dann mit der Signalverarbeitungseinheit verbunden ist.

10. Vollständig wegwerfbarer EKG-Recorder nach einem der Ansprüche 1 bis 9, wobei die Signalverarbeitungseinheit eine Antenne (34) umfasst, ein Begrenzungselement (217) in der Hülle bereitgestellt ist und das Begrenzungselement verwendet wird, um die Position der Antenne zu begrenzen.

11. Vollständig wegwerfbarer EKG-Recorder nach einem der Ansprüche 1 bis 10, wobei der vollständig wegwerfbare EKG-Recorder ferner eine Isolierzuglasche (4) umfasst; wobei sich die Isolierzuglasche bevorzugt von der Innenseite der Hülle zu der Außenseite der Hülle erstreckt; wobei die Isolierzuglasche herausziehbar ist; wobei, bevor sie herausgezogen wird, die Isolierzuglasche dazu konfiguriert ist, die elektrische Verbindung, zum Beispiel die elektrische Verbindung mit oder in der Signalverarbeitungseinheit, zu unterbrechen oder zu zerstören.

12. Vollständig wegwerfbarer EKG-Recorder nach Anspruch 11, wobei elektrische Verbindung mit einem Federkontakt oder -kontakten verwendet wird und die elektrische Verbindung mit einem Federkontakt oder -kontakten durch die Isolierzuglasche getrennt ist.

13. Vollständig wegwerfbarer EKG-Recorder nach einem der Ansprüche 1 bis 12, wobei von oben nach unten die elektrische Schicht eine Substratschicht (122b), eine elektrische Pfadschicht (122c), eine Isolationsschicht (122d) und eine klebrige Schicht (122e) aufweist.

14. EKG-Überwachungssystem, umfassend den EKG-Recorder nach einem der Ansprüche 1 bis 13 und einen Server, der dazu konfiguriert ist, EKG-Daten von dem EKG-Recorder zu empfangen, wobei der Server dazu konfiguriert ist, die EKG-Daten nach dem Empfangen der EKG-Daten von dem EKG-Recorder zu verarbeiten.

## Revendications

1. Enregistreur d'électrocardiographe, ECG, entièrement jetable, comprenant
un patch souple (1), conçu pour être fixé à la surface d'un corps humain, le patch souple comprenant une couche électrique (12), la couche électrique comprenant un trajet électrique souple (122) et au moins deux électrodes d'ECG (121), ledit trajet électrique souple étant connecté auxdites au moins deux électrodes d'ECG,
une coque (2), formant un espace fermé ;
une unité de traitement de signal (3), ladite unité de traitement de signal étant située dans l'espace fermé ;
ledit trajet électrique souple possédant une extrémité de connexion (122a),
ledit patch souple étant fixé sur un côté inférieur de la coque tourné vers la peau,
ledit côté inférieur comportant une première ouverture (216) pour le passage de l'extrémité de connexion ; et
ladite extrémité de connexion du trajet électrique souple s'étendant vers le haut dans l'espace fermé à l'intérieur de la coque, de manière à être connectée à l'unité de traitement de signal.

2. Enregistreur d'ECG entièrement jetable selon la revendication 1,
ledit patch souple étant pourvu d'une couche de patch adhésif (11), et ladite couche de patch adhésif comportant une seconde ouverture (11c) correspondant à la première ouverture ; ladite couche de patch adhésif possédant une surface inférieure (11a) et une surface supérieure (11b), une partie du trajet électrique souple étant étalée sur la surface inférieure, et une autre partie du trajet électrique souple étant courbée de manière à entrer dans l'espace fermé à travers la seconde ouverture et la première ouverture.

3. Enregistreur d'ECG entièrement jetable selon l'une quelconque des revendications 1 à 2, ladite coque comprenant une première partie de coque (21) et une seconde partie de coque (22).

4. Enregistreur d'ECG entièrement jetable selon la revendication 3,
ladite première partie de coque et ladite seconde partie de coque étant des parties de coque distinctes, qui sont reliées de manière inamovible l'une à l'autre de manière à former l'espace fermé.

5. Enregistreur d'ECG entièrement jetable selon l'une quelconque des revendications 1 à 4, une feuille de renforcement (122f) étant pourvue au niveau de l'extrémité de connexion du trajet électrique souple.

6. Enregistreur d'ECG entièrement jetable selon l'une quelconque des revendications 1 à 5, ledit patch souple étant pourvu d'une première couche adhésive (13), et ladite première couche adhésive recouvrant la première ouverture, de manière à assurer l'espace fermé dans la coque ; de préférence, ladite première couche adhésive recouvrant au moins une partie du trajet électrique souple mais ne recouvrant aucune électrode.

7. Enregistreur d'ECG entièrement jetable selon l'une quelconque des revendications 1 à 6, ledit patch souple étant pourvu d'une seconde couche adhésive (14) qui est conçue pour adhérer à la coque, de sorte que la surface inférieure de la coque et le patch souple soient reliés de manière fixe.

8. Enregistreur d'ECG entièrement jetable selon l'une quelconque des revendications 3 à 7 lorsqu'elle cite la revendication 3, une bague d'étanchéité souple (215) étant agencée au niveau de la surface correspondante de la seconde partie de coque et de la première partie de coque.

9. Enregistreur d'ECG entièrement jetable selon l'une quelconque des revendications 1 à 8, après que l'extrémité de connexion du trajet électrique souple pénètre dans l'espace fermé dans la coque, ladite extrémité de connexion étant pliée plusieurs fois puis connectée à l'unité de traitement de signal.

10. Enregistreur d'ECG entièrement jetable selon l'une quelconque des revendications 1 à 9, ladite unité de traitement de signal comprenant une antenne (34), un élément de limitation (217) étant pourvu dans la coque, et ledit élément de limitation étant utilisé pour limiter la position de l'antenne.

11. Enregistreur d'ECG entièrement jetable selon l'une quelconque des revendications 1 à 10, ledit enregistreur d'ECG entièrement jetable comprenant en outre une languette de traction isolante (4) ;
ladite languette de traction isolante s'étendant de préférence de l'intérieur de la coque à l'extérieur de la coque ; ladite languette de traction isolante pouvant être retirée ; avant d'être retirée, ladite languette de traction isolante étant conçue pour interrompre ou rompre la connexion électrique, par exemple la connexion électrique vers ou dans l'unité de traitement de signal.

12. Enregistreur d'ECG entièrement jetable selon la revendication 11, une connexion électrique avec un ou plusieurs contacts à ressort étant utilisée, et ladite connexion électrique avec un ou plusieurs contacts à ressort étant coupée par la languette de traction isolante.

13. Enregistreur d'ECG entièrement jetable selon l'une quelconque des revendications 1 à 12, ladite couche électrique comportant, de haut en bas, une couche de substrat (122b), une couche de trajet électrique (122c), une couche d'isolation (122d) et une couche collante (122e).

14. Système de surveillance d'ECG, comprenant l'enregistreur d'ECG selon l'une quelconque des revendications 1 à 13, et un serveur, configuré pour recevoir des données d'ECG en provenance de l'enregistreur d'ECG, ledit serveur étant configuré pour traiter les données d'ECG après avoir reçu les données d'ECG de l'enregistreur d'ECG.
